# DEMANDE DE BREVET EUROPEEN

(11) **EP 1 051 968 A2**
(43) Date de publication de la demande: **15.11.2000**
(21) Numéro de dépôt: 00401054.2
(22) Date de dépôt: 14.04.2000
(51) Int. Cl.: A61K 7/48, A61K 7/032

(54) **Composition cosmétique comprenant une dispersion aqueuse de polymère filmogène et d'organopolysiloxane**

(30) Priorité: 11.05.1999 FR 9905999
(71) Demandeur: L'OREAL, 75008 Paris (FR)
(72) Inventeur: Jager Lezer, Nathalie, 92340 Bourg la Reine (FR)
(74) Mandataire: Kromer, Christophe

(57) **Abrégé**

L'invention a pour objet une composition à appliquer sur la peau et les phanères comprenant une dispersion aqueuse de particules de polymère filmogène et une suspension aqueuse de particules d'organopolysiloxane solide élastomérique au moins partiellement réticulé.

L'invention concerne aussi un procédé de soin ou de maquillage des matières kératiniques consistant à appliquer la composition sur les matières kératiniques.

La composition permet d'obtenir un film présentant une bonne tenue et résistant à l'eau, de bon confort.

## Description

La présente invention a pour objet une composition contenant une dispersion aqueuse de particules de polymère filmogène et de particules d'organopolysiloxane, destinée en particulier au domaine cosmétique. Plus précisément, l'invention se rapporte à une composition pour le soin et le maquillage des matières kératiniques comme la peau, y compris les lèvres et le cuir chevelu, les ongles, les cheveux, les cils et les sourcils d'êtres humains.

Cette composition peut se présenter sous la forme de mascara, d'eye-liner, de produit pour les lèvres, de fard à joues ou à paupières, de fond de teint, de produit de maquillage du corps, de produit anti-cernes, de produit pour les ongles, de composition de protection solaire, de coloration de la peau, de produit de soin de la peau ou des phanères.

Les produits de maquillage ou de soin de la peau ou des lèvres d'êtres humains comme les eye-liners, les fonds de teints, les rouges à lèvres, ou bien encore des cils comme les mascaras, sont habituellement appliqués sous la forme d'une mince couche uniforme conduisant à la formation d'un film.

Il est connu des documents US-3639572 et US-A-4423031 des compositions de maquillage de la peau, notamment d'eye-liner, ou des cils comprenant une dispersion aqueuse de polymère filmogène. Le film obtenu avec ces compositions aqueuses ne présente pas toujours une bonne résistance à l'eau et le film, au contact de l'eau pendant la baignade ou la douche par exemple, se désagrège en partie en s'effritant ou bien encore en diffusant. L'effritement et la diffusion du film engendre une perte sensible de l'intensité de la couleur du maquillage, obligeant la consommatrice à renouveler l'application du produit de maquillage. La diffusion du film forme, par ailleurs, une auréole très inesthétique autour de la zone maquillée (yeux notamment). Les larmes et la transpiration provoquent également ces mêmes inconvénients. Ceci est notamment le cas pour un eye-liner.

De plus, ces compositions présentent également l'inconvénient de transférer, c'est-à-dire de se déposer au moins en partie, en laissant des traces sur certains supports avec lesquels elles peuvent être mises en contact, et notamment un tissu (vêtements, serviettes, mouchoirs), la peau (doigts) ou bien encore un verre, une tasse ou une cigarette pour les produits pour les lèvres. Il s'ensuit une diminution, voire une disparition, du maquillage nécessitant de renouveler régulièrement l'application de la composition de maquillage. Par ailleurs, l'apparition de traces inacceptables, notamment sur les cols de chemisier, peut écarter certaines femmes de l'utilisation de ce type de maquillage.

En outre, le film obtenu avec ces compositions peut être rêche au toucher ou ne pas être suffisamment souple, laissant une sensation d'inconfort et de gêne à l'utilisatrice.

Le but de la présente invention est de disposer d'une composition de maquillage ou de soin des matières kératiniques s'appliquant facilement et conduisant à un film présentant une bonne tenue et résistant à l'eau, de bon confort.

Les inventeurs ont découvert que l'utilisation d'une suspension aqueuse de particules d'organopolysiloxane solide élastomérique dans une composition comprenant une dispersion aqueuse de particules de polymère filmogène permettait d'obtenir une composition conduisant à la formation d'un film parfaitement résistant à l'eau. Le film obtenu présente également des propriétés de non transfert et présente un aspect mat, conférant ainsi à la composition des propriétés matifiantes. La composition est confortable à l'application, de bon étalement, douce et non collante au toucher

De façon plus précise, la présente invention a pour objet une composition à appliquer sur la peau et les phanères comprenant une dispersion aqueuse de particules de polymère filmogène , caractérisée par le fait qu'elle comprend une suspension aqueuse de particules d'organopolysiloxane solide élastomérique au moins partiellement réticulé.

L'invention a aussi pour objet un procédé cosmétique de maquillage ou de traitement non thérapeutique des matières kératiniques consistant à appliquer sur les matières kératiniques une composition telle que définie précédemment.

L'invention a également pour objet l'utilisation, dans une composition cosmétique ou pour la fabrication d'une composition topique, d'une dispersion aqueuse de polymère filmogène et d'une suspension aqueuse de particules solides d'organopolysiloxane élastomérique au moins partiellement réticulé pour obtenir un film résistant à l'eau et/ou ne transférant pas et/ou confortable et/ou mat.

Par « élastomérique » on entend un matériau souple, déformable ayant des propriétés viscoélastiques et notamment la consistance d'une éponge ou d'une sphère souple. Son modèle d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanes élastomériques conformes à l'invention sont partiellement ou totalement réticulés et de structure tridimensionnelle, et se présentent sous forme de poudre dispersée dans de l'eau.

Les organopolysiloxanes élastomériques selon l'invention peuvent être choisis parmi les polymères réticulés décrits dans la demande JP-A-10/175816. Selon cette demande, ils sont obtenus par réaction d'addition et de réticulation, en présence d'un catalyseur notamment du type platine, d'au moins :
- (a) un organopolysiloxane (i) ayant au moins deux groupes vinyliques en position α-ω de la chaîne siliconée par molécule ; et
- (b) un organosiloxane (ii) ayant au moins un atome d'hydrogène lié à un atome de silicium par molécule.

En particulier, l'organopolysiloxane (I) est choisi parmi les polydiméthylsiloxanes et est plus spécifiquement un α-ω-diméthylvinyl polydiméthylsiloxane.

Les organopolysiloxanes élastomériques de la composition selon l'invention se présente avantageusement sous forme de suspension aqueuse. Cette suspension peut être notamment obtenues comme suit :
- (a) mélange de l'organopolysiloxane (i) et de l'organosiloxane (ii) ;
- (b) ajout de la phase aqueuse contenant un émulsifiant au mélange de l'étape (a) ;
- (c) émulsification de la phase aqueuse et dudit mélange ;
- (d) ajout d'eau chaude à l'émulsion de la phase (c) ; et
- (e) polymérisation de l'organopolysiloxane (i) et de l'organosiloxane (ii) en émulsion en présence d'un catalyseur de platine.

L'eau est avantageusement ajoutée à une température de 40-60°C. Après l'étape (e), il est possible de sécher les particules obtenues, pour en évaporer toute ou partie de l'eau piégée.

Les organopolysiloxanes de la composition de l'invention sont par exemple ceux commercialisés sous les noms BY 29-122 et BY 29-119 par la société Dow-Corning Toray. On peut aussi utiliser un mélange de ces produits commerciaux.

De façon préférentielle, la poudre d'organopolysiloxane élastomérique est présente dans la composition en une teneur en matières sèches allant de 0,5 à 65% en poids, par rapport au poids total de la composition, de préférence de 3 à 45% en poids, et mieux de 5 % à 30 % en poids, et en tout état de cause en une quantité suffisante pour augmenter la résistance à l'eau de la composition.

En particulier, les particules d'organopolysiloxane élastomérique (en matière active) peuvent avoir une taille allant de 0,1 à 500 µm et mieux de 3 à 200 µm. Ces particules peuvent être sphériques, plates ou amorphes avec, de préférence, une forme sphérique.

Selon l'invention, les particules d'organopolysiloxane sont présentes dans la composition sous forme de suspension aqueuse ; elles sont donc directement en suspension dans l'eau. Cette dernière peut être ajoutée à toute composition quelle que soit sa forme galénique..

Ces particules d'organopolysiloxane, pour se disperser de façon stable dans l'eau, peuvent être associées à un ou plusieurs tensioactifs non ioniques, cationiques ou anioniques de HLB ≥ 8. De préférence, l'étape c) est réalisée en présence d'un tensio-actif non-ionique.

La proportion de tensioactifs est de préférence de 0,1 à 20 parties en poids pour 100 parties en poids de la composition d'organopolysiloxane élastomérique, et mieux, de 0,5 à 10 parties en poids (cf. description du document JP-A-10/175816).

La composition selon l'invention contient, en outre, un polymère filmogène se présentant sous la forme de particules en dispersion aqueuse, connue généralement sous le nom de latex ou pseudolatex.

Dans la présente demande, on entend par "polymère filmogène", un polymère apte à former à lui seul ou en présence d'un agent auxiliaire de filmification, un film isolable.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).
Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

On utilise de préférence des polymères filmogènes radicalaires anioniques, c'est-à-dire des polymères ayant au moins un monomère à groupement acide.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₂₀, de préférence en C₁-C₈, des (méth)acrylates d'aryle, en particulier d'aryle en C₆-C₁₀, des (méth)acrylates d'hydroxyalkyle, en particulier d'hydroxyalkyle en C₂-C₆ .
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide et le N-t-octyl acrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme polymère filmogène acrylique utilisable selon l'invention, on peut citer ceux vendus sous les dénominations NEOCRYL XK-90^{®}, NEOCRYL A-1070^{®}, NEOCRYL A-1090^{®}, NEOCRYL BT-62^{®}, NEOCRYL A-1079^{®}, NEOCRYL A-523 ^{®} par la société ZENECA, DOW LATEX 432^{®} par la société DOW CHEMICAL.

Parmi les polycondensats filmogènes, on peut également citer les polyuréthanes, les polyesters, les polyesters amides, les polyesters à chaîne grasse, les polyamides, et les résines époxyesters.

Les polyuréthanes peuvent être choisis parmi les polyuréthannes anioniques, cationiques, non-ioniques ou amphotères, les polyuréthannes-acryliques, les polyuréthannes-polyvinylpirrolidones, les polyester-polyuréthannes, les polyéther-polyuréthannes, les polyurées, les polyurée-polyuréthannes, et leurs mélanges. Le polyuréthanne filmogène peut être, par exemple, un copolymère polyuréthanne, polyurée-uréthanne, ou polyurée, aliphatique, cycloaliphatique ou aromatique, comportant seule ou en mélange :
- au moins une séquence d'origine polyester aliphatique et/ou cycloaliphatique et/ou aromatique, et/ou,
- au moins une séquence siliconée, ramifiée ou non, par exemple polydiméthylsiloxane ou polyméthylphénylsiloxane, et/ou
- au moins une séquence comportant des groupes fluorés.

Les polyuréthannes filmogènes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.

Comme polyuréthanne filmogène utilisable selon l'invention on peut utiliser ceux commercialisés sous les dénominations NEOREZ R-981^{®}, NEOREZ R-974^{®} par la société ZENECA, les AVALURE UR-405^{®}, AVALURE UR-410^{®}, AVALURE UR-425^{®}, AVALURE UR-450^{®}, SANCURE 875^{®}, SANCURE 861^{®}, SANCURE 878^{®}, SANCURE 2060^{®} par la société GOODRICH, IMPRANIL 85^{®} par la société BAYER, AQUAMERE H-1511^{®} par la société HYDROMER.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphta-lènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.
On préfère utiliser dans les compositions objet de l'invention des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ par la société Eastman Chemical Products.

Les polyesters peuvent notamment comprendre un monomère porteur d'un chromophore, comme ceux décrit dans la demande WO 98/26753.

Les polymères d'origine naturelle, éventuellement modifiés, peuvent être choisis parmi la résine shellac, la gomme de sandaraque, les dammars, les élémis, les copals, les polymères cellulosiques insolubles dans l'eau, et leurs mélanges.

On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides.

La dispersion comprenant un ou plusieurs polymères filmogènes peut être préparée par l'homme du métier sur base de ses connaissances générales.

La taille des particules de polymères en dispersion aqueuse peut aller de 10 à 500 nm, et de préférence de 20 à 300 nm.

Le polymère filmogène en dispersion aqueuse peut être présent dans la composition selon l'invention en une teneur en matières sèches allant de 0,1 % à 60 % en poids par rapport au poids total de la composition, de préférence de 0,1 % à 40% en poids, et mieux de 0,1 % à 20 % en poids. L'adjonction de l'organopolysiloxane élastomérique permet en particulier d'augmenter de façon notable la résistance à l'eau d'une composition contenant un faible taux de latex ou pseudolatex (à savoir inférieur à 30 %, voire inférieur à 10 % en poids de matières sèches de polymère filmogène, par rapport au poids total de la composition).

La composition selon l'invention peut comprendre un agent auxiliaire de filmification favorisant la formation d'un film avec les particules du polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

Le milieu aqueux (ou phase aqueuse) de la composition peut être constitué essentiellement d'eau. Il peut comprendre également un mélange d'eau et de solvant miscible à l'eau comme les monoalcools inférieurs ayant de 1 à 5 atomes de carbone, les glycols ayant de 2 à 8 atomes de carbone, les cétones en C₃-C₄, les aldéhydes en C₂-C₄. Il représente, en pratique, de 5 % à 99,4 % en poids, par rapport au poids total de la composition.

La composition peut également comprendre au moins une matière colorante comme les composés pulvérulents et/ou les colorants hydrosolubles et/ou les colorants liposolubles, par exemple à raison de 0,01 à 50 % du poids total de la composition. Les composés pulvérulents peuvent être choisis parmi les pigments et/ou les nacres et/ou les charges habituellement utilisés dans les compositions cosmétiques ou dermatologiques. Avantageusement, les composés pulvérulents représentent de 0,1 à 25 % du poids total de la composition et mieux de 1 à 20 %.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être choisies parmi celles bien connues de l'homme du métier et couramment utilisées dans les compositions cosmétiques.

Parmi les colorants hydrosolubles, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, leurs mélanges.

Parmi les colorants liposolubles, on peut citer le rouge Soudan, le DC Red 17, le DC Green 6, le β-carotène, l'huile de soja, le brun Soudan, le DC Yellow, 11, le DC Violet 2, le DC orange 5, le jaune quinoléine.

La composition peut comprendre, en outre, un ou plusieurs actifs notamment cosmétiques ou dermatologiques, tels que les agents hydratants, les vitamines, les acides gras essentiels, les protéines, les céramides, les filtres solaires, les agents anti-radicaux libres, les agents anti-inflamatoires. Bien entendu, l'homme du métier veillera à choisir ce ou ces éventuels composés complémentaires, et/ou leur quantité, de manière telles que les propriétés avantageuses de la composition pour l'utilisation selon l'invention ne soient pas, ou substantiellement pas, altérées par l'adjonction envisagée. Ces actifs peuvent être par exemple utilisés en une teneur allant de 0,001 % à 20 % en poids, par rapport au poids total de la composition.

La composition selon l'invention peut, de plus, comprendre selon le type d'application envisagée, les constituants classiquement utilisés dans les domaines considérés, qui sont présents en une quantité appropriée à la forme galénique souhaitée.

La composition peut contenir, en outre, tout additif usuellement utilisés dans de telles compositions tels que les épaississants, les parfums, les conservateurs, les tensioactifs différents de ceux de la suspension d'organopolysiloxane, les huiles, les cires, les antioxydants.

Avantageusement, la composition de l'invention peut contenir comme additifs un ou plusieurs gélifiants de phase aqueuse. Parmi les gélifiants de phase aqueuse utilisables selon l'invention, on peut citer les gélifiants cellulosiques hydrosolubles tels que l'hydroxyéthylcellulose, la méthylcellulose, l'hydroxypropylcellulose et la carboxyméthylcellulose ; la gomme de guar ; la gomme de guar quaternisée ; les gommes de guar non-ioniques comprenant des groupements hydroxyalkyle en C₁-C₆ ; les gommes de xanthane, de caroube, de scléroglucane, de gellane, de rhamsan, de karoya ; les alginates, la maltodextrine, l'amidon et ses dérivés, l'acide hyaluronique et ses sels ; les argiles et notamment les montmorillonites, les hectorites ou bentones, les laponites ; les polymères à groupement carboxylique comme les acides polyacryliques réticulés au moins partiellement neutralisé tels que les «Carbopol » ou «Carbomer» de la Société Goodrich (Carbomer 980 par exemple neutralisé par de la triéthanolamine -TEA en abréviation-) ; les polymères poly(métha)crylates de glycéryle ; la polyvinylpyrrolidone ; l'alcool polyvinylique ; les polymères et les copolymères réticulés d'acrylamide ; les homopolymères réticulés de chlorure de méthacryloyloxyéthyltriméthylammonium ; les polyuréthanes associatifs.

Selon l'invention, le gélifiant de phase aqueuse est de préférence choisi parmi la gomme de xanthane, les argiles (bentone ou laponite), les polyuréthanes associatifs, les épaississants cellulosiques, notamment l'hydroxyéthyl cellulose, et les acides polyacryliques réticulés au moins partiellement neutralisés.

La composition peut se présenter sous forme de gel, de lotion, de stick, d'émulsion eau-dans-huile, huile-dans-eau, cire-dans-eau, eau-dans-cire, d'émulsion multiple, de dispersion vésiculaire contenant des lipides ioniques et/ou non ioniques.

Ces compositions à application topique peuvent constituer notamment une composition cosmétique, dermatologique, hygiénique ou pharmaceutique de protection, de soin ou de traitement de la peau, notamment pour le visage, pour le cou, pour les mains ou pour le corps (par exemple crème de soin, gel solaire, gel corporel), une composition de maquillage, de composition de protection solaire ou une composition de bronzage artificiel.

La composition de maquillage peut être notamment un mascara, un eye-liner, un produit pour les lèvres (rouge à lèvres), un fard à paupières ou à joues, un produit anti-cernes, un fond de teint, un produit pour les ongles (vernis-à-ongles ou base de soin), un produit de maquillage du corps du type tatouage provisoire ou semi-permanent.

L'invention est illustrée plus en détail dans les exemples suivants.

### Exemples 1 à 8 comparatifs :

On a préparé 4 fards à paupières selon l'invention (exemples 1 à 4) et 4 fards à paupières ne faisant pas partie de l'invention (exemples 1' à 4') ayant les compositions suivantes :
Exemples 1 à 4 :
   - Organopolysiloxane en suspension aqueuse à 63 % de matières sèches (BY29-119 de la société Dow Corning) 20,16 g MA
   - Hydroxypropyl cellulose 2 g
   - Polymère filmogène en dispersion aqueuse 2,45 g MA
   - Glycérine 1 g
   - Nacre 5 g
   - Eau qsp 100 g
Exemples 1' à 4' :
   - Hydroxypropyl cellulose 2 g
   - Polymère filmogène en dispersion aqueuse 2,45 g MA
   - Glycérine 1 g
   - Nacre 5 g
   - Eau qsp 100 g

On a utilisé les dispersions aqueuse de polymère filmogène suivantes :
Exemples 1 et 1' : Copolymère Polyvinyl pyrrolidone à 15 % MA (AQUAMERE H-1511 de la société HYDROMERE)
Exemples 2 et 2' : Copolymère acrylique à 60 % MA (NEOCRYL A-523 de la société ZENECA)
Exemples 3 et 3' : Polyuréthane à 49 % MA (AVALURE UR-425 de la société GOODRICH)
Exemples 4 et 4' : Polyuréthane-polyéther à 27 % MA (SANCURE 2060 de la société GOODRICH)

Pour chaque composition, on a appliqué sur une plaque de verre une couche de 200 µm d'épaisseur avant séchage puis on a laissé séché pendant une heure à température ambiante. On a fait couler un filet d'eau sur le film sec et on a mesuré le temps (en seconde) nécessaire pour commencer à dégrader le film.

On a obtenu les résultats suivants :

| **Exemple** | **Temps** |
|---|---|
| 1 | 240 |
| 1' | 70 |
| 2 | 180 |
| 2' | 90 |
| 3 | 70 |
| 3' | 30 |
| 4 | 60 |
| 4' | 25 |

On constate que les films des exemples 1 à 4 selon l'invention ont une résistance à l'eau améliorée par rapport à celle des films des exemples 1' à 4' ne contenant pas l'organopolysiloxane en suspension aqueuse.

## Revendications

1. Composition à appliquer sur la peau et les phanères comprenant une dispersion aqueuse de particules de polymère filmogène, caractérisée par le fait qu'elle comprend une suspension aqueuse de particules d'organopolysiloxane solide élastomérique au moins partiellement réticulé.

2. Composition selon la revendication 1, caractérisée par le fait que l'organopolysiloxane élastomérique est obtenu par réaction d'addition et de réticulation, en présence d'un catalyseur, d'au moins :
- un organopolysiloxane (i) ayant au moins deux groupements vinyliques en position α-ω de la chaîne siliconée par molécule ; et
- d'un organosiloxane (ii) ayant au moins un atome d'hydrogène lié à un atome de silicium par molécule.

3. Composition selon la revendication 1 ou 2, caractérisée par le fait que l'organopolysiloxane (I) est choisi parmi les polydiméthylsiloxanes.

4. Composition selon l'une quelconque des revendications précédentes, caractérisée par le fait que l'organopolysiloxane est un α-ω-diméthylvinyl polydiméthylsiloxane.

5. Composition selon l'une quelconque des revendications précédentes, caractérisée par le fait que la suspension aqueuse de particules d'organopolysiloxane élastomérique est obtenue selon les étapes suivantes :
- (a) mélange de l'organopolysiloxane (i) et de l'organosiloxane (ii) ;
- (b) ajout de la phase aqueuse contenant un émulsifiant au mélange de l'étape (a) ;
- (c) émulsification de la phase aqueuse et dudit mélange ;
- (d) ajout d'eau chaude à l'émulsion de la phase (c) ; et
- (e) polymérisation de l'organopolysiloxane (i) et de l'organosiloxane (ii) en émulsion en présence d'un catalyseur de platine.

6. Composition selon la revendication 5, caractérisée par le fait que l'étape (c) est réalisée en présence d'un tensioactif non-ionique.

7. Composition selon l'une quelconque des revendications précédentes, caractérisée par le fait que les particules d'organopolysiloxane élastomérique ont une taille allant de 0,1 à 500 µm et mieux de 3 à 200 µm.

8. Composition selon l'une quelconque des revendications précédentes, caractérisée par le fait que l'organopolysiloxane élastomérique est présent en une teneur en matières sèches allant de 0,5 % à 65 % en poids, par rapport au poids total de la composition, et mieux de 3 % à 45 % en poids.

9. Composition selon l'une quelconque des revendications précédentes, caractérisée par le fait que le polymère filmogène est choisi dans le groupe formé par les polymères radicalaires, les polycondensats, les polymères d'origine naturelle et leurs mélanges.

10. Composition selon l'une quelconque des revendications précédentes, caractérisée par le fait que le polymère filmogène est choisi parmi les polymères vinyliques, les polyuréthanes, les polyesters.

11. Composition selon l'une quelconque des revendications précédentes, caractérisée par le fait que le polymère fiilmogène en dispersion aqueuse est présent en une teneur en matières sèches allant de 0,1 % à 60 % en poids, par rapport au poids total de la composition.

12. Composition selon l'une quelconque des revendications précédentes, caractérisée par le fait qu'elle comprend au moins un solvant miscible à l'eau.

13. Composition selon la revendication 12, caractérisée par le fait que que le solvant miscible à l'eau est choisi dans le groupe formé par les monoalcools inférieurs ayant de 1 à 5 atomes de carbone, les glycols ayant de 2 à 8 atomes de carbone, les cétones en C₃-C₄, les aldéhydes en C₂-C₄.

14. Composition selon l'une quelconque des revendications précédentes, caractérisée par le fait qu'elle comprend au moins un additif choisi dans le groupe formé par les épaississants, les parfums, les conservateurs, les tensioactifs différents de ceux de la suspension d'organopolysiloxanes, les huiles, les cires, les plastifiants, les agents de coalescence, les matières colorantes, les charges, les antioxydants, les actifs cosmétiques ou dermatologiques.

15. Composition selon l'une quelconque des revendications précédentes, caractérisée par le fait qu'elle se présente sous la forme d'un mascara, d'un eye-liner, d'un produit pour les lèvres, d'un fard à paupières ou à joues, d'un produit anti-cernes, d'un fond de teint, d'un produit de maquillage du corps, d'un produit pour les ongles, de soin ou de traitement des matières kératiniques, de composition de protection solaire, de composition de bronzage artificiel.

16. Procédé cosmétique de maquillage des matières kératiniques, caractérisé par le fait que l'on applique sur les matières kératiniques une composition selon l'une quelconque des revendications 1 à 15.

17. Procédé de traitement non thérapeutique des matières kératiniques, caractérisé par le fait que l'on applique sur les matières kératiniques une composition selon l'une quelconque des revendications 1 à 15.

18. Utilisation, dans une composition cosmétique ou pour la fabrication d'une composition topique, d'une dispersion aqueuse de polymère filmogène et d'une suspension aqueuse de particules solides d'organopolysiloxane élastomérique au moins partiellement réticulé pour obtenir un film résistant à l'eau et/ou ne transférant pas et/ou confortable et/ou mat.
